# EUROPEAN PATENT APPLICATION

(11) **EP 4 154 918 A2**
(43) Date of publication of application: **29.03.2023**
(21) Application number: 22193490.4
(22) Date of filing: 01.09.2022
(51) Int. Cl.: A61L 2/18

(54) **BIOFILM PREVENTION**

(30) Priority: 03.09.2021 US 202163240411 P; 24.08.2022 US 202217894424
(71) Applicant: Kohler Co., Kohler, WI 53044 (US)
(72) Inventor: GARG, Yachna, 122001 Gurgaon (IN); KWACZ, Jason, Kohler, 53044 (US); TARPLEE, Jennifer, Sheboygan Falls, 53085 (US); KURU, William, Plymouth, 53073 (US); DENZIN, Peter, Glenbeulah, 53023 (US); SMITH, Andrew, Sheboygan, 53081 (US); WHITFIELD, Robin, Kohler, 53044 (US)
(74) Representative: Barker Brettell LLP

(57) **Abstract**

A biofilm mitigation apparatus includes a supply container, a disinfectant generator, a power source, and a delivery tube. The supply container is configured to store one or more materials for a formation of a disinfectant. The disinfectant generator is configured to generate the disinfectant from the one or more materials from the supply container. The power source is configured to provide electrical power to the disinfectant generator. The delivery tube is configured to transport the disinfectant from the disinfectant generator to a biofilm.

## Description

This application claims priority benefit of US Provisional Application No. 63/240,411 filed September 3, 2021, and US Application No. 17/894,424, filed on August 24, 2022, which are hereby incorporated by reference in their entirety.

### FIELD

The present application relates generally to biofilm prevention in plumbing fixtures.

### BACKGROUND

A biofilm is a group of one or more types of microorganisms that can grow on a variety surfaces. Example microorganisms that form biofilms include bacteria, fungi and protists. Biofilms may form on bodies of water, plant tissue, animal tissue, teeth, underwater, and inside of other living organisms. Potentially, biofilms may form, generally, on any water exposed areas. Plumbing fixtures, fittings and water supply systems and installations for washing, showering, bathing and similar devices may include water exposed areas.

### BRIEF DESCRIPTION OF THE DRAWINGS

Exemplary embodiments are described herein with reference to the following drawings, according to an exemplary embodiment.
FIG. 1 illustrates an example biofilm prevention device removably coupled to a lavatory or sink.
FIG. 2 illustrates an example biofilm prevention device coupled to a plumbing system.
FIG. 3 illustrates another example biofilm prevention device.
FIG. 4 illustrates another example biofilm prevention device.
FIG. 5 illustrates an example biofilm prevention device coupled to multiple appliances.
FIG. 6 illustrates another example biofilm prevention device coupled to a plumbing system.
FIG. 7 illustrates an example controller for any off the biofilm prevention systems.
FIG. 8 illustrates a flow chart for the controller of FIG. 6.
FIG. 9 illustrates an example internal automated biofilm prevention device for a plumbing system.
FIG. 10 illustrates an example transport track for the internal automated biofilm prevention device.
FIG. 11 illustrates another example transport track for the internal automated biofilm prevention device.
FIG. 12 illustrates an example robotic scrubber for the internal automated biofilm prevention device.
FIG. 13 illustrates an example robotic scrubber for the internal automated biofilm prevention device.
FIG. 14 illustrates an example magnetic stopper for a drain.
FIG. 15 illustrates an example mechanically operated disposable sleeve for a drain.
FIG. 16 illustrates an example automated disposable sleeve for a drain.
FIG. 17 illustrates an example rotatable P-trap assembly.
FIG. 18 illustrates a side view of the rotatable P-trap assembly.
FIG. 19 illustrates another example rotatable P-trap assembly.
FIG. 20 illustrates an example foam fractionation device for a drain.
FIG. 21 illustrates an example ultrasonic cavitation device.
FIG. 22 illustrates an example dielectric barrier discharge device.
FIG. 23 illustrates an example dielectric barrier discharge device.
FIG. 24 illustrates a flow chart for the controller of the barrier discharge device.

### DETAILED DESCRIPTION

The following embodiments include devices and techniques for the reduction, mitigation, or prevention of biofilms in plumbing devices. The plumbing devices may include pipes, faucets, bathtubs, showers, water softeners, water heaters, toilets or other devices. The term "plumbing fixture" refers to an apparatus that is connected to a plumbing system of a house, building or another structure. The term "bathroom fixture" may more specifically refer to individual types of plumbing fixtures found in the bathroom. The term "kitchen fixture" may more specifically refer to individual types of plumbing fixtures found in the kitchen.

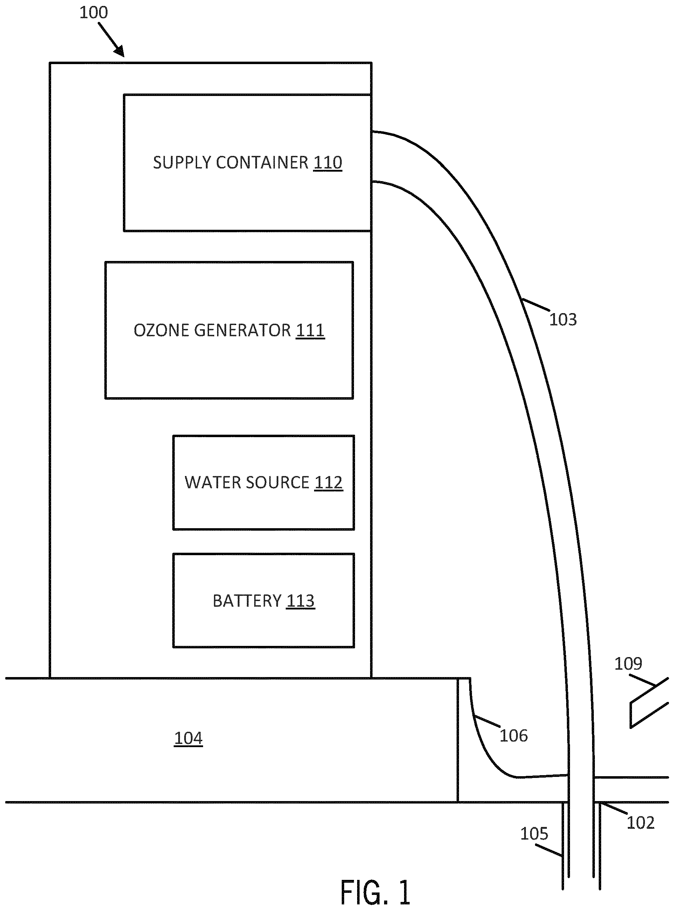

FIG. 1 illustrates an example biofilm prevention device 100 removably coupled to a lavatory or sink 106, or another plumbing device. The biofilm prevention device 100 may include a supply container 110, a disinfectant generator (e.g., ozone generator 111 or hydrogen peroxide generator), a water source 112, a power source (e.g., battery 113 or a power outlet), and a delivery tube 103. The sink 106 may be mounted to a countertop 104 and include a nozzle or faucet 109. The sink 106 may include a drain 102 coupled to a tail pipe 105, which may be referred to as a drain pipe, extended downstream of the drain 102. Additional, fewer or different components may be included. As shown in other embodiments, the biofilm prevention device 100 may be connected to the plumbing device at any point along the plumbing downstream of the plumbing device. The biofilm prevention device 100 may be integrated with the plumbing device. A faucet, bathtub, shower, water softener, water heater, or a toilet may include the biofilm prevention device 100.

The supply container 110 is configured to store one or more materials for the formation of disinfectant. The disinfectant generator is configured to generate disinfectant from the one or more materials from the supply container 110. The one or more materials for the formation of the disinfectant may include water, gas (e.g., oxygen gas, nitrogen gas), air, and/or other materials. The disinfectant, in whole or in part, may include hydrogen peroxide or silver ions. The disinfectant generator may include a pump, a charger, or another powered device powered by the power supply. The disinfectant generator may receive an input from the water source 112. The water may be mixed with the disinfectant.

In some embodiments, the disinfectant generator is an ozone generator 111, which generates ozone gas or O³. The ozone gas may react with or oxidize bacteria, organic material, or other pollutants. In some examples, water is provided from the supply container 110 and the ozone generator 111 creates ozone gas from the water. The ozone gas may be generated using a corona charger or corona discharge (e.g., spark discharge). The ozone may be generated from air and/or water. A high voltage may be applied to the corona charger to cause corona discharge to electrically breakdown water or air in the vicinity of the corona charger. The corona charger may include a charge or voltage that exceeds the dielectric strength of the water and/or air, breaking down the water and/or air into one or more components and ozone gas.

The power source is configured to provide electrical power to the disinfectant generator. The power source may be an AC source (e.g., the biofilm prevention device 100 may include a power cord). The power source may be a DC source such as the battery 113 in FIG. 1.

In some embodiments, in addition or in the alternative of the disinfectant generator, an induced biology generate may introduce microorganisms to the plumbing fixture (e.g., drain 102 and/or tail pipe 105). The microorganisms may release an enzyme that may degrade the biofilm. Different enzymes may be included, such as glycosidases, proteases and deoxyribonuclease (DNase). The enzymes may degrade different components in an extracellular polymeric substance (EPS) such as specifically polysaccharides, proteins and eDNA. This destabilizes the biofilm by altering the biofilm architecture and increasing the penetration of antibiotics. Induction can be done by adding chemicals, detergents, radiations, temperature etc. By using enzyme detergents that contain a mixture of protease, D-Nase I, amylase and cellulose, it can destabilize the biofilm EPS and improve the efficiency of biofilm removal.

The delivery tube 103 is configured to transport the disinfectant from the disinfectant generator to a biofilm. The delivery tube 103 may include a hose made of rubber or another flexible material. The delivery tube 103 may include a screw type connector on either or both ends of the tube 103. One connector may connect to the biofilm prevention device 100, and the other end may connect to the drain 102, the tail pipe 105, or to a plumbing system as described below. The delivery tube 103 provides the disinfectant to the drain 102 or elsewhere in the plumbing device. The disinfectant may degrade or slow the growth of the biofilm. The disinfectant may cause the biofilm to be removed from the plumbing device.

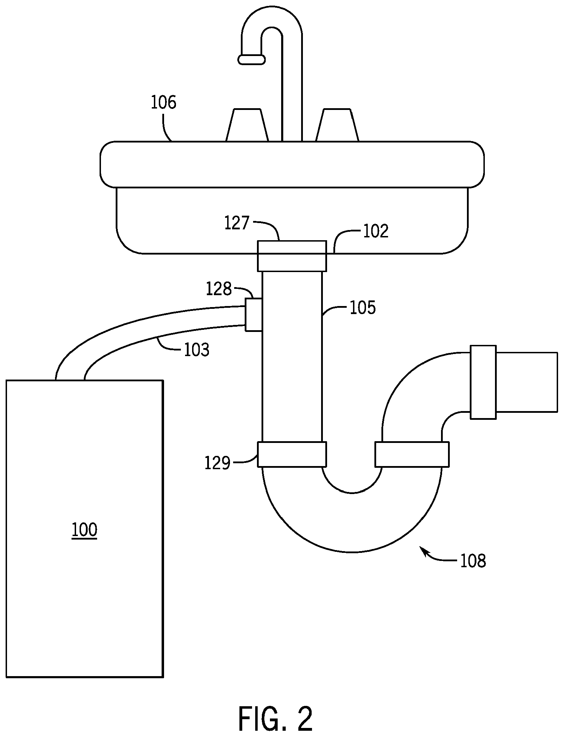

In examples similar to FIG. 1, the delivery tube 103 is configured to be coupled to an upstream side of the tail pipe 105 through the drain 102. As an alternative or in addition, the delivery tube 103 may be configured to be coupled to a downstream side of the tail pipe 105 through a plumbing system 108, in examples similar to that of FIG. 2.

FIG. 2 illustrates an example biofilm prevention device 100 coupled to a plumbing system 108 for a lavatory or sink 106. The biofilm prevention device 100 of this embodiment may be connected to any plumbing device or integrated therein. The biofilm prevention device 100 may include any of the components described above (e.g., the supply container 110, the disinfectant generator (e.g., ozone generator 111), the water source 112, the power source (e.g., battery 113)). The biofilm prevention device 100 is connected to the plumbing system 10 via the delivery tube 103, for example, at the tail pipe 105 extending from the drain 102. Additional, fewer or different components may be included.

The delivery tube 103 may include a valve 128 configured to turn on or off the disinfectant provided into the tail pipe 105 for the prevention of biofilm formation. The valve 128 may include a mechanical knob or lever for operation of the valve. The valve 128 may include a motor or solenoid operated by a controller (e.g., controller 400 described herein).

Similarly, the plumbing system 108 may include a valve for closing off the tail pipe 108 or the P-trap during the biofilm prevention process. The valve 129 may include a mechanical knob or lever for operation of the valve 129. The valve 129 may include a motor or solenoid operated by a controller (e.g., controller 400 described herein). The controller may close the valve 129 in response to operation of the biofilm prevention device 100 or the user input to start the biofilm prevention device 100. The valve 129 prevents the disinfectant or other substance from escape downstream during the biofilm prevention process.

In addition, the drain 102 may be closed off during the biofilm prevention process. That is, the user may insert the tube 103 into the drain, initiate the biofilm prevention process, and then place a cap 127 on the drain. Alternatively, such a cap 127 may be automatically applied or removed by the controller according to a timer.

Using in any of these examples, the controller may perform a disinfectant cycle having multiple stages. The process may start and end in any stage and be performed in any order. In an example order, a first stage includes normal operation of the appliance, that is, water is drained without the biofilm prevention process. In some examples, the controller is not involved in causing the first stage. In other examples, the controller turns on a pump to provide water to the sink 106.

In a second stage, the biofilm prevention device 100 provides the disinfectant to the appliance. The controller may open a valve that causes the disinfectant and water to exit the supply container 110 and be provided to the plumbing device through the delivery tube 103. Alternatively or in the addition, the controller may operate a pump to cause the water to exist the supply container 110 and be provided to the plumbing device through the delivery tube 103.

In a third stage, a cap is applied to the drain and/or the plumbing system is closed to enclose the disinfectant within the drain. For example, the controller may close a valve that blocks the drain 105. The valve may be integrated with the cap 127. The controller may actuate a solenoid or motor for closing off the drain 105.

In a fourth stage, the cap 127 and/or a valve in the plumbing system is opened. As in the third stage, the controller may actuate a solenoid, a motor, or other device for opening passage to the drain 105.

A different time period may be applied to each stage. In one example, the second, third, and fourth stages elapse in approximately 20 to 30 minutes. For example, the controller may perform the second stage, then start a timer for a predetermined time and proceed to perform the third stage when the timer reaches a predetermined duration. Likewise, the controller may perform the third stage, then start a timer for a predetermined time and proceed to perform the fourth stage when the timer reaches a predetermined duration.

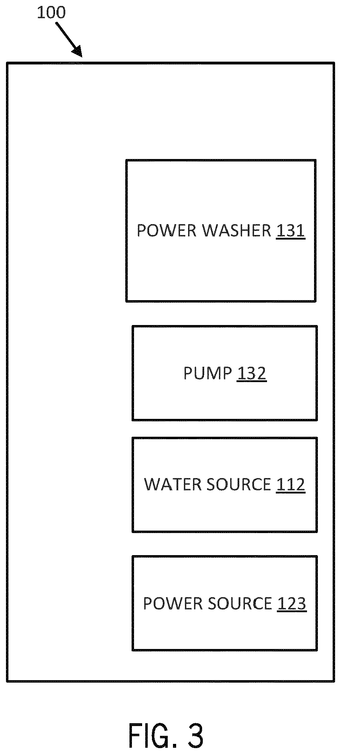

FIG. 3 illustrates another example biofilm prevention device 100. In this embodiment, the biofilm prevention device 100 includes power washer 131 (or alternatively a sand blaster), which may internally or externally include a pump 132, a water source 112, and a power source 123. Additional, different or fewer components may be included.

The power source 123 may include a battery (e.g., battery 113). The power source 123 may include an engine using a fuels source (e.g., gasoline, propane, compressed natural gas). The power source 123 may include an air tank or other source of compressed air.

The biofilm prevention device 100 may be placed in any of the configurations described herein including the delivery tube 103 is configured to be coupled to an upstream side of the tail pipe 105 through the drain 102 or coupled to a downstream side of the tail pipe 105 through a plumbing system 108. The biofilm prevention device 100 may be a modular or portable unit near the drain 102, places the delivery into the drain 102 or onto an openable port on the plumbing system 108.

After installation, the user may turn on the biofilm prevention device 100 to start the power washer 131. The pump 132 of power washer 131 is powered via the power source 123 to generate water pressure to provide water from water source 112 into the drain 102 or the tail pipe 105. High pressure water, even without ozone or another substance, may prevent the formation of biofilms. In some embodiments, ozone or another material may be combined with water in this embodiment.

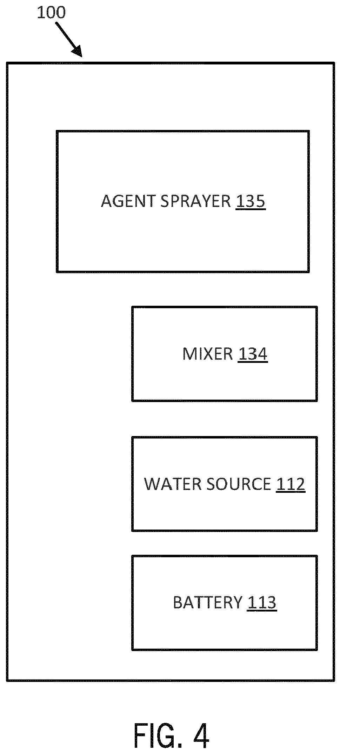

FIG. 4 illustrates another example biofilm prevention device 100 including an agent sprayer 135. The agent may be a chemical such as a solution including one or more of hydrogen peroxide, hypochlorous acid, iodine, bromine, chloramine, chlorine dioxide, peracetic acid, quaternary ammonium, tetraacetyl ethylenediamine, phenolic, isopropyl alcohol, sodium carbonate, peroxyhydrate, tetraacetyl ethylenediamine, ethanol, sodium hypochlorite, octanoic acid, or sodium chlorite. The mixer 134 may mix water from the water source 112 for dispensing by the agent sprayer 135 into the drain 102 or the plumbing system 108 according to any of the examples herein, including manual and/or automated operation and/or the disinfectant cycle.

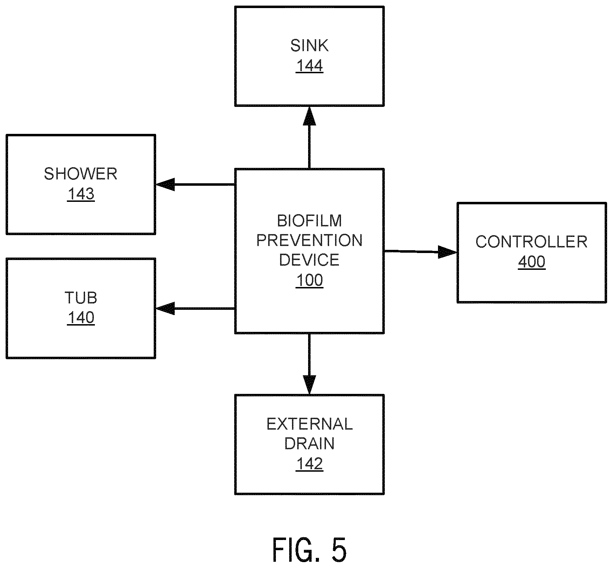

FIG. 5 illustrates an example biofilm prevention device 100 coupled to multiple appliances (e.g., a bathtub 140 or a hot tub, shower 143, a sink 144, and/or an external drain 142). The biofilm prevention device 100 may integrated in a central location. The central location may be within a wall, connected to a plumbing system for the multiple appliances. The central location may be in a basement or utility room, for example, and may be coupled or otherwise integrated with a water heater, a water softener, or a grey water system. Additional, different, or other features may be included.

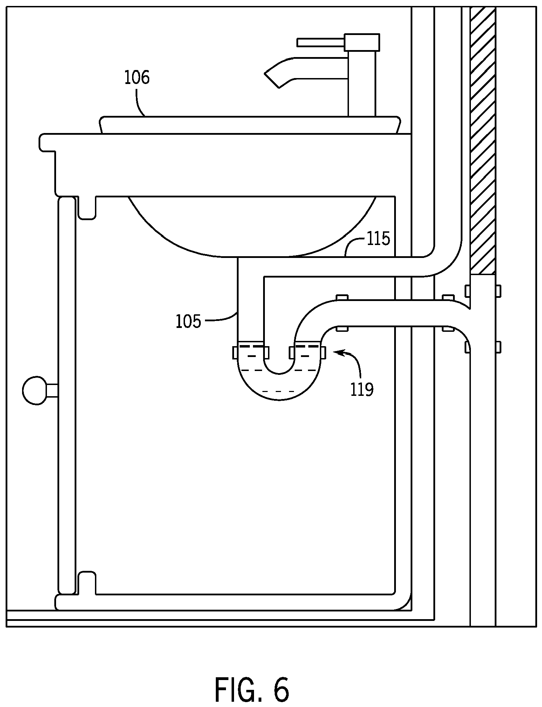

FIG. 6 provides an example for the plumbing system, including a biofilm prevention pipe 115, for one such appliance as a sink 106 coupled to a tail pipe 105 and a P-trap 119. For example, the biofilm prevention device 100 may be connected to a bathtub 140 or a hot tub, shower 143, a sink 144, and/or an external drain 142. The external drain 142 may be in a floor (e.g., for a basement or a laundry room) or a swimming pool. The shower 143 may include a shower head configured to spray water and a drain. The bathtub 140 may include a faucet and/or one or more jets and a partially enclosed container including a drain. Additional, different or fewer components may be included.

The plumbing system 108 described herein may be coupled to any combination of the appliances of FIG. 5. The plumbing system 108 is connected to the appliances and transports the disinfectant to the appliances.

The plumbing system 108 may include at least one valve configured to release the disinfectant to at least one of the appliances. For example, a valve may include in a path of the disinfectant between the biofilm prevention device 100 and each of the appliances.

A controller (e.g., controller 400 described below) may be configured to control each of the valves according to a schedule. In one example, the valves are controlled in response to a user input. In another example, one or more of the valves are controlled in response to the analysis of sensor data. The controller 400 is configured to receive user selections or sensor data and determine one or more valve commands in response. The sensors 321 may be the same or different types of sensors.

The sensor data may be received by a variety of sensors. The sensor may include a flow sensor, an environment sensor, a water level sensor, or another type of sensor. The flow sensor may measure an amount of water that flows through the drain 102 or that is dispensed by the nozzle 109. The controller 400 may determine whether a threshold amount of water has been dispensed, and in response, begin the biofilm prevention process. The environment sensor may include a temperature sensor, a humidity sensor, and/or other types of sensors configured to detect the condition of the drain 102. The controller 400 may determine whether conditions have been present for biofilm formation (e.g., for a predetermined amount of time), and in response, begin the biofilm prevention process. The water level sensor may determine whether water has backed up into the drain 102 (e.g., for a predetermined amount of time), and in response, begin the biofilm prevention process.

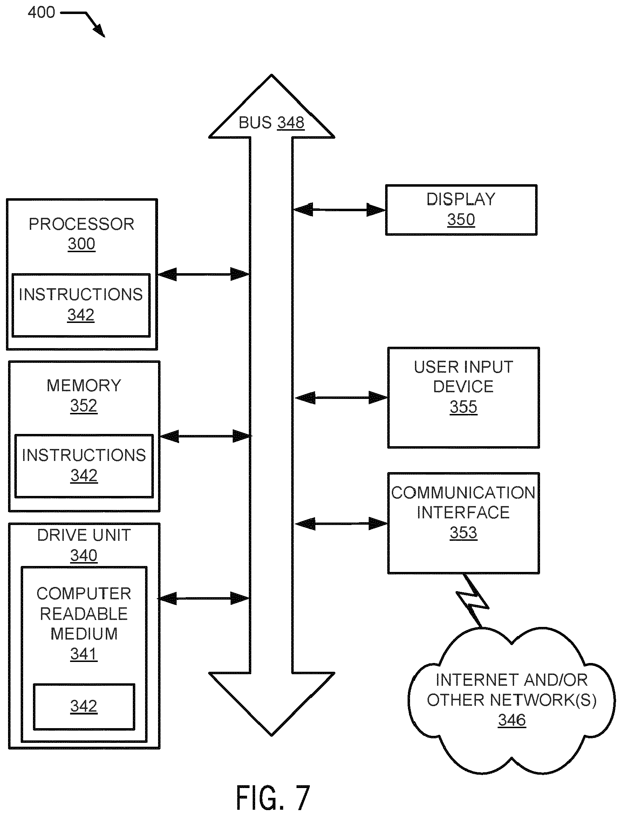

FIG. 7 illustrates an example controller for the biofilm prevention system. The controller 400 may include a processor 300, a memory 352, and a communication interface 353 for interfacing with devices or to the internet and/or other networks 346. In addition to the communication interface 353, a sensor interface may be configured to receive data from the sensors described herein or data from any source for analyzing water properties or the operation of the appliances described herein. The components of the control system 400 may communicate using bus 348. The control system 400 may be connected to a workstation or another external device (e.g., control panel) and/or a database for receiving user inputs, system characteristics, and any of the values described herein.

Optionally, the control system 400 may include an input device 355 and/or a sensing circuit in communication with any of the sensors. The sensing circuit receives sensor measurements from as described above. The input device 355 may include a switch, a touchscreen coupled to or integrated with the mirror, a keyboard, a microphone for voice inputs, a camera for gesture inputs, and/or another mechanism.

Optionally, the control system 400 may include a drive unit 340 for receiving and reading non-transitory computer media 341 having instructions 342. Additional, different, or fewer components may be included. The processor 300 is configured to perform instructions 342 stored in memory 352 for executing the algorithms described herein. A display 350 may be supported by any of the components described herein. The display 350 may be combined with the user input device 355.

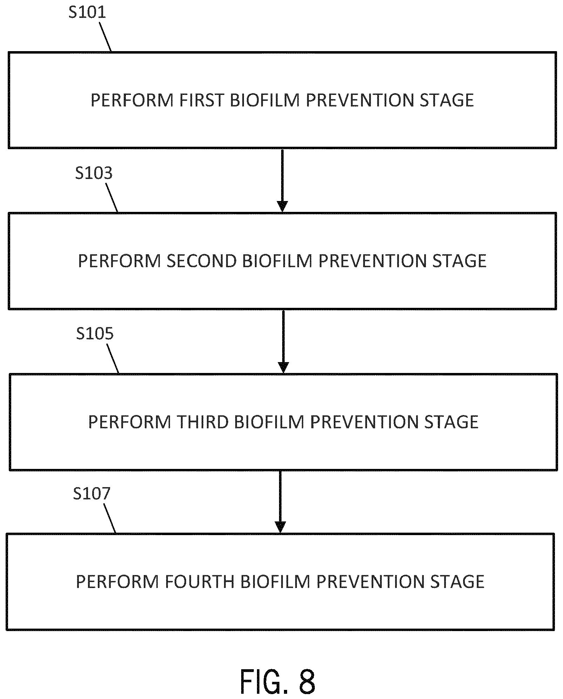

FIG. 8 illustrates a flow chart for the controller of the biofilm prevention system. The acts of the flow chart may be performed by any combination of the control system 400, the network device, or the server. Portions of one or more acts may be performed by the appliance. Additional, different, or fewer acts may be included.

At act S101, the controller 400 may perform a first stage including normal operation of the appliance, that is, water is drained without the biofilm prevention process. For example, the plumbing device may operate using one or more functions of the plumbing device. In the case of a sink of lavatory 106, the faucet (valve) may be operated to turn on and off, adjust the volume, and/or adjust the temperature of water that is dispensed from the faucet.

At act S103, the controller 400 may perform a second stage, the biofilm prevention device 100 provides the disinfectant to the appliance. The controller 400 may opening the outlet of the biofilm prevention device 100. The controller 400 may activate the disinfectant generator to create the disinfectant. The controller 400 may open the valve for the delivery tube 103 to dispense the disinfectant into the plumbing device.

At act S105, the controller 400 may perform a third stage in which a cap or cover is applied (closed), through a solenoid or other mechanical device, to the drain and/or the plumbing system to enclose the disinfectant within the drain. At act S105, the controller 400 may perform a fourth stage, wherein the cap and/or the plumbing system is opened.

Act S 107 may be performed by the reverse action of act S 103, for example, the reverse action of the controller 400 to open the cap and/or the plumbing system. The controller 400 may start a timer to measure the amount of time that the disinfectant is within the plumbing device. Different types of disinfectants may be assigned to different amounts of time. The controller 400 may access the predetermined time for the timer based on the disinfectant that is used. When the controller 400 determines that the disinfectant 400 has been exposed to the drain or other components of the plumbing device, the controller 400 causes act S107 to open the drain.

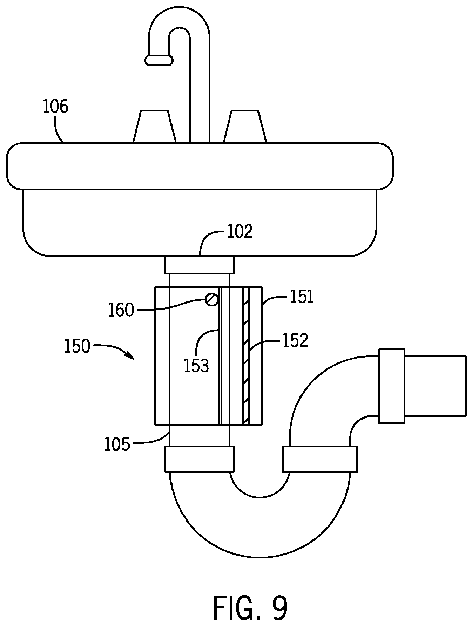

FIG. 9 illustrates an example internal automated biofilm prevention device for a plumbing system. The biofilm prevention device may include a robot system 150 including at least one robotic scrubber 160 corresponding to a robotic scrubber area 153, at least one movable guide member 152, and a drive mechanism. The robot system 150 may be comprised of internal components that are inside of the tail pipe 105 and external components that are outside of the tail pipe 105. The external components may be enclosed by a housing 151. Additional, different or fewer components may be included.

A robotic scrubber area 153 is configured to be cleaned by the at least one robotic scrubber 160. For example, the robotic scrubber area 153 may have a different diameter than the remaining areas of the tail pipe 105. The robotic scrubber area 153 may include a different type of surface (e.g., textured) than the remaining areas of the tail pipe 105.

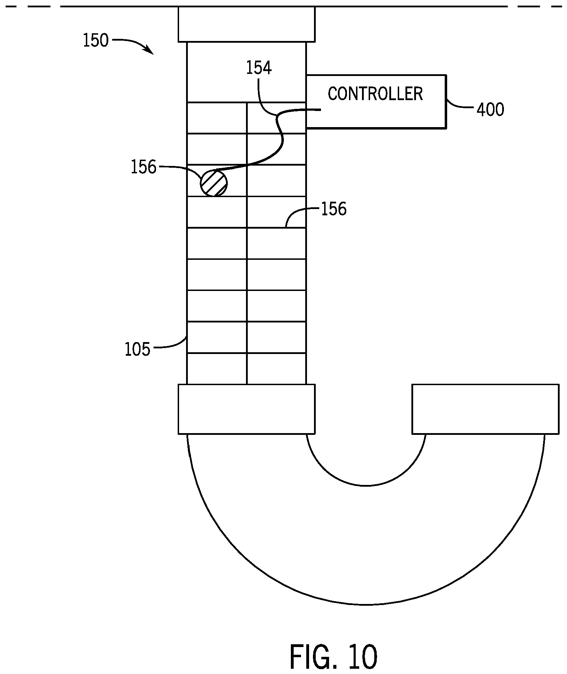

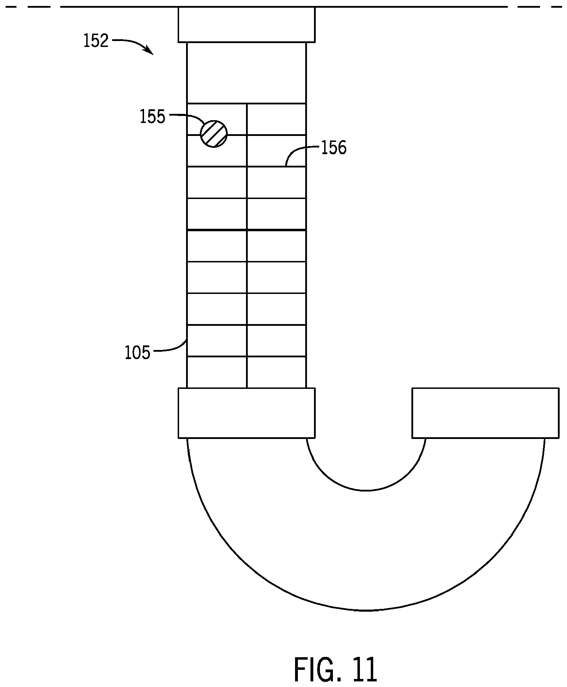

FIGS. 10 and 11 illustrate the internal automated biofilm prevention device. The robotic scrubber 160 may be supported on an inside of the drain pipe 105 by a movable guide member 152. The movable guide member is in proximity (e.g., in contact with or within a small distance range) to the outside of the drain pipe and supported on an outside of the drain pipe and configured to guide the robotic scrubber 160 inside of the pipe. For example, a transport track 156 may support a carrier 155. The carrier 155 may be a magnet, an electromagnetic, or any device configured to apply a force to the robotic scrubber 160 through the tail pipe. The carrier 155 may include one or more groves, ridges, or channels to travel along the transport track 156 in one or more directions around the outside of the tail pipe 105.

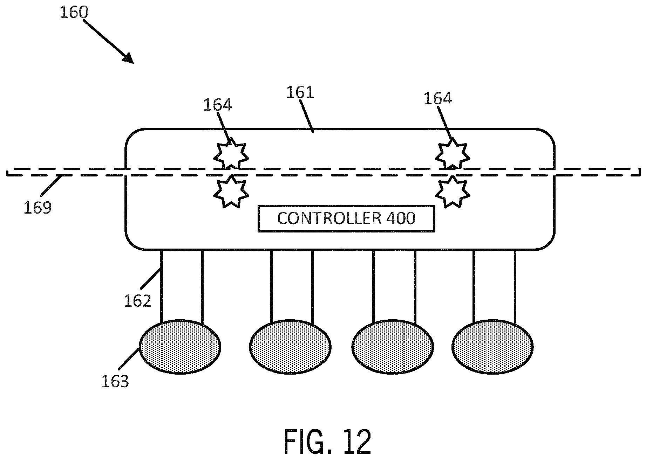

A controller (e.g., controller 400) may be included on the outside of the drain pipe (external controller embodiments) or incorporated with the robotic scrubber 160 (internal controller embodiments). For the external controller embodiments, as illustrated in FIG. 10, the controller 400 may be mounted on a side of the drain pipe 105 or under the sink 106. For the internal controller embodiments, for example, as illustrated in FIG. 12, the controller 400 may in incorporated in the body of the robotic scrubber 160.

In one alternative, not shown, a manual handle is connected to the drive mechanism. The manual handle allows the user to move the movable guide member 152 in the predetermined path. The manual handle may be a crank or lever and the drive mechanism may include a gear or cable that is coupled to the movable guide member 152.

The controller 400 is configured to generate a command for the drive mechanism to move the movable guide member in the predetermined path. In the external controller embodiments, the movable guide member 152 moves across the transport track 156. The predetermined path may be set by the paths of the transport track 156. In the internal controller embodiments, the predetermined path may be set by the controller 400.

The drive mechanism is configured to move the movable guide member in a predetermined path. The predetermined path may include at least one circumferential direction path, which is parallel to the circumference of the tail pipe 105. The circumferential direction is perpendicular to the direction of flow through the tail pipe 105. The predetermined path may include at least one horizontal direction that is parallel to the direction of flow through the tail pipe 105.

The controller 400 may both receive instructions from a user or external device and send the commands to the movable guide member 152 and/or the robotic scrubber 160. The commands from the user may be received locally to the robot system 150 via a button, touchscreen, or keypad mounted or electrically coupled to the robot system 150. The commands from the user may be received from a mobile device via wireless signal (e.g., cellular, Bluetooth, Wifi, etc.).

In some examples, the controller 400 may generate the commands for the movable guide member 152 and/or the robotic scrubber 160 based on sensor data. The sensor data may describe a presence or a quantity of biofilm or a condition associated with biofilm as described in other embodiments.

In FIG. 10 the controller 400 is connected to the carrier 155 via a cable or tether 154. The carrier 155 may be supported by the transport track 156 on the outside of the tail pipe 105. The tether 154 between the movable guide member 152 and the drive mechanism may include one or more electrical wires carrying the commands from the controller and power to the robot scrubber 160.

In FIG. 11, the controller 400 is incorporated into the robot scrubber 160. In this embodiment, the controller 400 may provide commands to move the carrier 155 (on the outside of the tail pipe 105) and/or the robot scrubber 160 (on the inside of the tail pipe 105) to mate or dock with a charging station. The charging station is coupled to the carrier 155 and configured to charge a battery of the robotic scrubber 160. The battery powers to drive system of the robot scrubber 160. The controller 400 may cause the robot scrubber 160 to move the charging station after a set amount of run time, at a time of day, or on a day of the week. The controller 400 may cause the robot scrubber 160 to move to the charging station in response to a battery level determined by a battery level sensor.

FIG. 12 illustrates an example robotic scrubber 160 for the internal automated biofilm prevention device. The robotic scrubber 160 may include a housing 161, at least one arm 162, at least one cleaning member 163, and at least one drive member 164. Additional, different, or fewer components are included.

The housing 161 may be a plastic or metal case that encloses the other components. The at least one arm 162 extends from the housing 161 to support the at least one cleaning member 163. The at least one cleaning member 163 may include an absorbent material, an abrasive material, a scouring pad, or another material suitable for scrubbing biofilm or other microorganisms from the interior of the tail pipe 105.

The at least one drive member 164 includes a gear, belt, or pulley that follows a path 169 in the scrubber area 153. The path 169 may be implemented by a groove, slot, or track in the tail pipe 105.

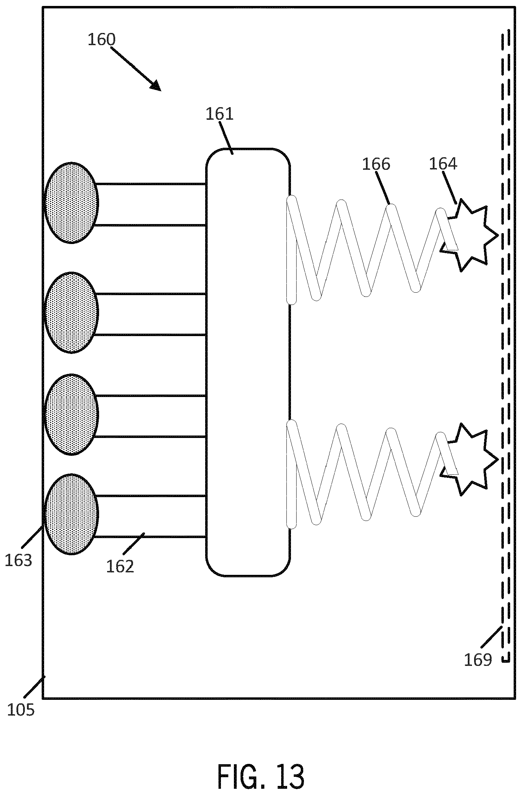

FIG. 13 illustrates another example robotic scrubber for the internal automated biofilm prevention device including a tensioning member 166. The tensioning member 166 may include a spring or another biasing member that can provide a force to support the robotic scrubber 160 in the tail pipe 105. The robotic scrubber 160 may be configured to expand out to be supported by the tail pipe 105.

In another example, the tail pipe 105 may include a screw path (an inclined path that includes multiple passes around the internal circumference of the tail pipe 105). The robotic scrubber 160 may following the screw path circumferentially to travel vertically within the tail pipe 105.

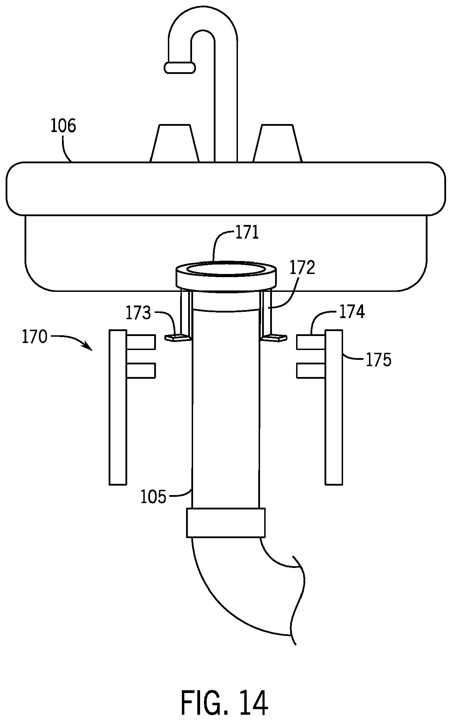

FIG. 14 illustrates an example magnetic stopper system or stopper assembly 170 for a drain of a sink 106. The stopper assembly 170 may be applied to any drain and/or any of the appliances described herein. The stopper assembly 170 may include a magnet guide 175, a stopper 171, and at least one magnet foot 173 supported by a rail 172. Additional, different, or fewer components may be included.

The stopper assembly 170 may also include a drive system for changing a position of the at least one driven magnet 174. The magnet guide 175 includes a track and at least one driven magnet 174 slidably engaged with the track of the magnet guide 175 and positioned to provide a magnet field. The track may include a slot that the magnet 174 can slide in. The magnet 174 may be positioned by a motor or solenoid operated by the controller 400. The motor or solenoid may be connected to the magnet via an additional drive element such as a gear or a belt to as the drive system.

The stopper 171 is configured to at least partially seal the drain 102. The at least one magnet foot 173 is coupled to the stopper and positioned in the magnetic field to move the stopper under a force applied by the magnet field. In some examples, the driven magnet 174 includes an upper driven magnet above the at least one magnet foot 173 and a lower driven magnet below the at least one magnetic foot 173. The at least one magnet foot 173 includes three magnetic feet spaced apart in a radial direction.

The drive system is configured to receive an input indicative of a stopper position. The controller 400 may be configured to generate the input for the stopper position. Examples stopper positions may be opened or closed. In the open position, the stopper 171 does not seal the drain such that water and other material may escape down the tail pipe 105. In the closed position, the stopper 171 seals the drain such that water and other material cannot escape down the tail pipe 105. Alternatively, the stopper 171 may partially seal the drain so that substantially all of the water is prevented from escaping down the tail pipe 105.

The controller 400 is configured to cause the drive system to move the at least one driven magnet 174 from the closed position to the open position where the stopper 171 is moved under the magnet field to unseal the drain.

The controller 400 may generate magnet commands to move the driven magnet 174 between the open and closed positions. The commands may be generated in response to an input (trigger) from a user interface, a timer, or a sensor. The user interface is electrically coupled to the controller 400. The input may be generated in response to the user interface. The timer may cause the input when a certain time or time of day has passed. The sensor may trigger the input to move the magnet 174 based a flow device or sensor, an environment sensor, a water level sensor, or another type of sensor. The flow sensor may measure an amount of water that flows through the drain 102 or that is dispensed by the nozzle 109. The controller 400 may determine whether a threshold amount of water has been dispensed, and in response, close the stopper assembly 170. The environment sensor may include a temperature sensor, a humidity sensor, and/or other types of sensors configured to detect the condition of the drain 102. The controller 400 may determine whether conditions have been present for biofilm formation (e.g., for a predetermined amount of time), and in response, close the stopper assembly 170. The water level sensor may determine whether water has backed up into the drain 102 (e.g., for a predetermined amount of time), and in response, close the stopper assembly 170.

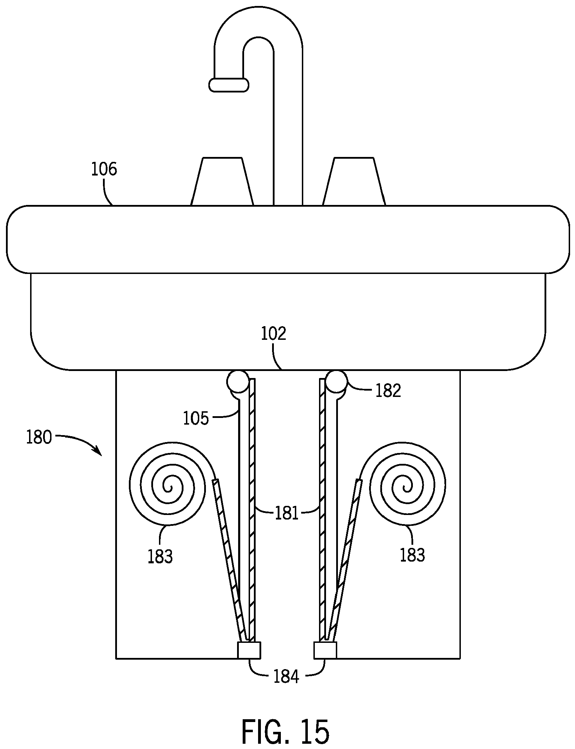

FIG. 15 illustrates an example disposable sleeve system 180 with a mechanically operated disposable sleeve 181 for a drain pipe 105. The disposable sleeve system 180 may include a compartment 183, a seal 184, and a locking ring 182. Additional, different, or fewer components may be included.

The compartment 183 is configured to house a length of disposable sleeve material outside off the drain pipe 105. The disposable sleeve material 181 may be plastic such as polyethylene. The compartment 183 may be an enclosure for a roll of the disposable sleeve material 181. The compartment 13 may include a cap or other removable portion to load or unload the disposable sleeve material 181.

The seal 184 is positioned at a first end of the drain pipe 105 and is configured to dispense the disposable material 181 into the drain pipe 105. The seal 184 allows a water tight, or partially water tight, path for the disposable sleeve material 181 to be advanced from outside of the drain pipe 105 to inside of the drain pipe 105. The portion of the disposable sleeve material 181 advanced into the drain pipe 105 may be referred to as a drain section. The drain section covers the inside of the drain pipe 105 to prevent the formation of biofilm.

The locking ring 182 is positioned at a second end of the drain pipe and configured to couple the drain section of the disposable sleeve material to the drain pipe 105. The disposable sleeve system 180 may also include a groove configured to receive and support a spent portion of the disposable sleeve material 181. The disposable sleeve system 180 may also include a cutting device configured to remove a portion of the disposable sleeve material 181. The cutting device may be incorporated with or near to the locking ring 182. Incorporated in the locking ring 182, or adjacent (below) the locking ring 182 may be a wiper so removing biological material (e.g., biofilm) from the disposable sleeve material 181.

The user may grip the portion of the disposable sleeve material 181 to advance the disposable sleeve material 181. To remove the old or spent material, the user may press the cutting device or push the disposable sleeve material 181 against the cutting device. The user may pull the disposable sleeve material 181 so that new disposable sleeve material is removed from the roll. The user may press the disposable material 181 against the locking ring 182 or otherwise close the locking ring 182 to hold the new disposable sleeve material 181 in place to further prevent the formation of biofilm.

As an alternative to this manual embodiment, the disposable sleeve system 180 may include an actuator to advance the disposable sleeve material. The actuator may be a handle or knob that receives a manual input. Alternatively, an electronic system may advance the disposable sleeve material 181.

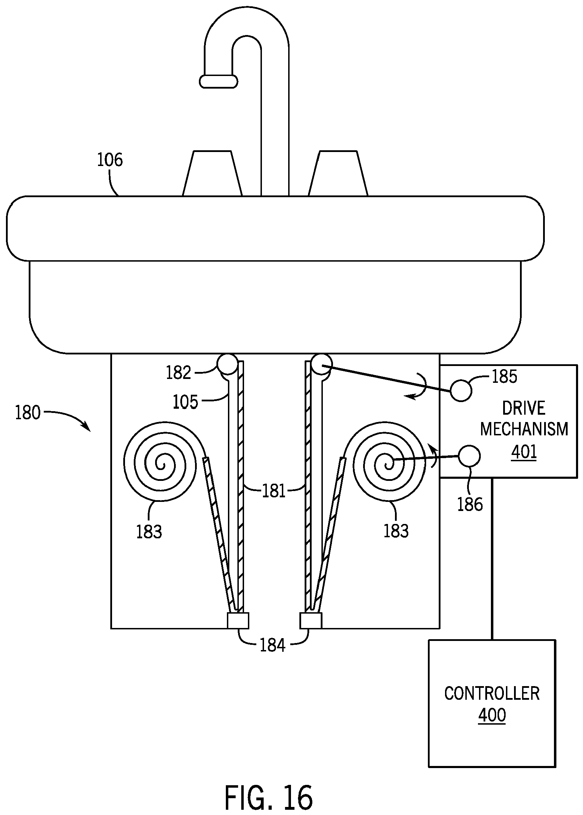

FIG. 16 illustrates an example automated disposable sleeve system 180 for the drain pipe 105. In addition to the components described with respect to FIG. 15, the disposable sleeve system 180 may include a drive mechanism 185 configured to advance the drain section of the disposable sleeve material into the drain pipe 105 and a controller 400 configured to generate a command for the drive mechanism to advance the drain section of the disposable sleeve material 181 into the drain pipe.

The controller 400 may be configured to control the actuation of the disposable sleeve material 181 according to one or more inputs. The input may be a schedule. The controller 400 may compare a current time to a predetermined time interval, time of day, or day of the week, and based on the comparison, advance the disposable sleeve material 181. The controller 400 may be configured to advance the disposable sleeve material based on sensor data. The sensor data describes an environment of the drain pipe 105, a biofilm formation in the drain pipe 105, or a usage of the drain pipe 105. The controller 400 is configured to receive user selections or sensor data and determine one or more disposable sleeve commands in response. The sensor data may be received by a variety of sensors. The sensor may include a flow sensor, an environment sensor, a water level sensor, or another type of sensor. The flow sensor may measure an amount of water that flows through the drain 102 or that is dispensed by the nozzle 109. The controller 400 may determine whether a threshold amount of water has been dispensed, and in response, begin the biofilm prevention process. The environment sensor may include a temperature sensor, a humidity sensor, and/or other types of sensors configured to detect the condition of the drain 102. The controller 400 may determine whether conditions have been present for biofilm formation (e.g., for a predetermined amount of time), and in response, begin the biofilm prevention process. The water level sensor may determine whether water has backed up into the drain 102 (e.g., for a predetermined amount of time), and in response, begin the biofilm prevention process.

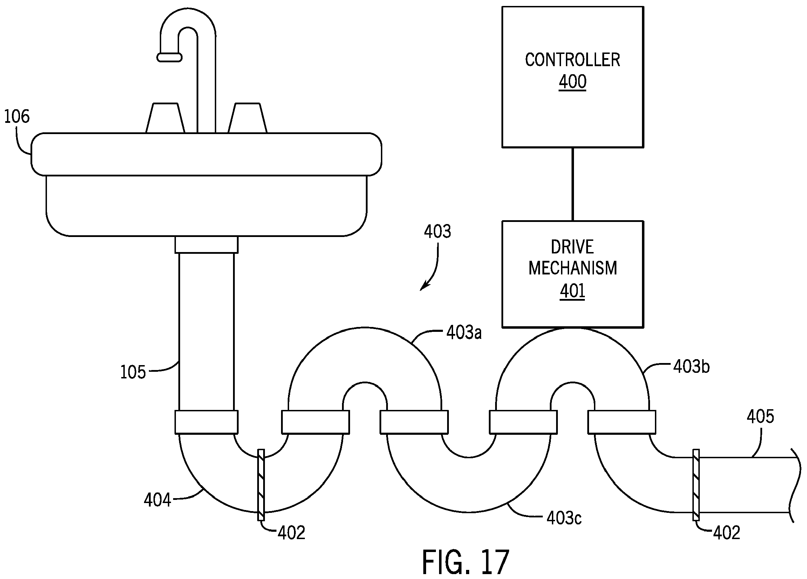

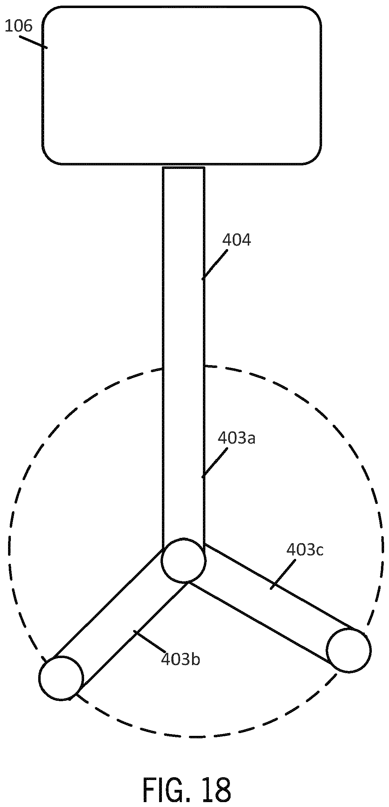

FIG. 17 illustrates an example rotatable P-trap assembly 403 for a plumbing system. FIG. 18 illustrates a side view of the rotatable P-trap assembly. The plumbing system may include a drain pipe 105, an upstream connector pipe 404, a downstream connector pipe 505 and the P-trap assembly 403. Additional, different, or fewer components may be included.

The rotatable P-trap may provide a sheering action to biofilm on other buildups that have formed inside the P-trap or along the plumbing system. The sheering action may disrupt growth or cause the biofilm to be separated and expelled into the drain to the sewer or septic system. The rotatable P-trap may also help to disrupt and remove clogs.

The upstream connector pipe 404 connects the drain pipe 105 to the P-trap assembly 403. The downstream connector pipe 405 connects the P-trap assembly 403 to the sewer or septic system of the house or building.

The P-trap assembly 403 includes one or more joints 402 between a plurality of trap sections. The joints 402 are rotatable joints between adjacent trap sections (e.g., between the first trap section and the second trap section). The joints 402 may include one or more bearings or other pivotable members.

The trap sections may include a first trap section 403a connected to the drain pipe 105 of the water consuming appliance (e.g., sink 106) and a second trap section 403b connected to the first trap section 403a and the connector pipe 404. The first trap section 403a includes a first trap at a first height and the second trap section 403b includes a second trap at a second height. Optionally, a third trap section 403c may be connected between the first trap section 403a and the second trap section 403b and/or a fourth trap section may be connected between the third trap section 403c and the second trap section 403b.

FIG. 18 illustrates an example of different heights of the trap sections. Each trap section may be a different height and/or placed at a different angle with respect to the first connector pipe 404 and the second connector pipe 40. The first trap holds water at a position of the drive mechanism where the second trap does not hold water.

In some examples, the P-trap assembly 403 is rotatable. That is the first trap, the second trap and/or other traps may be rotate to different heights and angles. The P-trap assembly 403 may be rotated through manual input such as a crank, handle, or knob.

A drive mechanism 401 is configured to move the first trap section 403a or the second trap section 403b relative to the drain pipe 105 or the connector pipe 404. The drive mechanism 401 may include a stepper motor or a solenoid. The drive mechanism 401 may be actuated based on a timer or a schedule. The drive mechanism 401 may be actuated based on sensor data.

A controller 400 is configured to generate a command to actuate the drive mechanism. The controller 400 may receive input data for the dynamic P-trap. The input data may include a schedule or sensor data. The controller 400 may compare a current time to a predetermined time interval, time of day, or day of the week, and based on the comparison, rotate the P-trap assembly 403. The sensor data describes an environment of the drain pipe, a biofilm formation in the drain pipe, or a usage of the drain pipe. The controller 400 is configured to receive user selections or sensor data and determine one or more rotation commands in response. The sensor data may be received by a variety of sensors. The sensor may include a flow sensor, an environment sensor, a water level sensor, or another type of sensor. The flow sensor may measure an amount of water that flows through the drain 102 or that is dispensed by the nozzle 109. The controller 400 may determine whether a threshold amount of water has been dispensed, and in response, begin the biofilm prevention process. The environment sensor may include a temperature sensor, a humidity sensor, and/or other types of sensors configured to detect the condition of the drain 102. The controller 400 may determine whether conditions have been present for biofilm formation (e.g., for a predetermined amount of time), and in response, begin the P-trap rotation. The water level sensor may determine whether water has backed up into the drain 102 (e.g., for a predetermined amount of time), and in response, begin the P-trap rotation.

The controller 400 may generate a rotation command for the dynamic P-trap. The rotation command may include an amount of rotation, a number of rotations, or a particular angle to move one or more of the traps of the P-trap assembly 403. For example, the rotation command includes a stepper motor angle. In another example, the rotation command may include a current level for an electromagnet in a solenoid that causes the P-trap assembly to rotate. The solenoid may ratchet a gear on an axle that supports the P-trap assembly. In other words, several alternatives are possible for the drive mechanism 401 to rotate the P-trap assembly 403 in response to the rotation command.

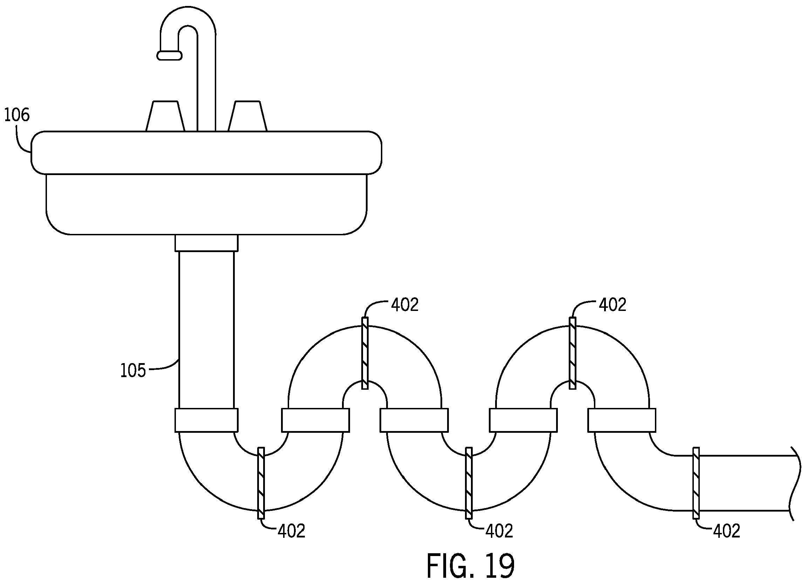

FIG. 19 illustrates another example rotatable P-trap assembly. In this example, four trap sections and five joints 402 are illustrated. Each of the joints 402 may be independently controlled by the controller 400. The two joints 402 on either side of the interior P-traps may be actuated by a solenoid or motor to rotate the P-trap.

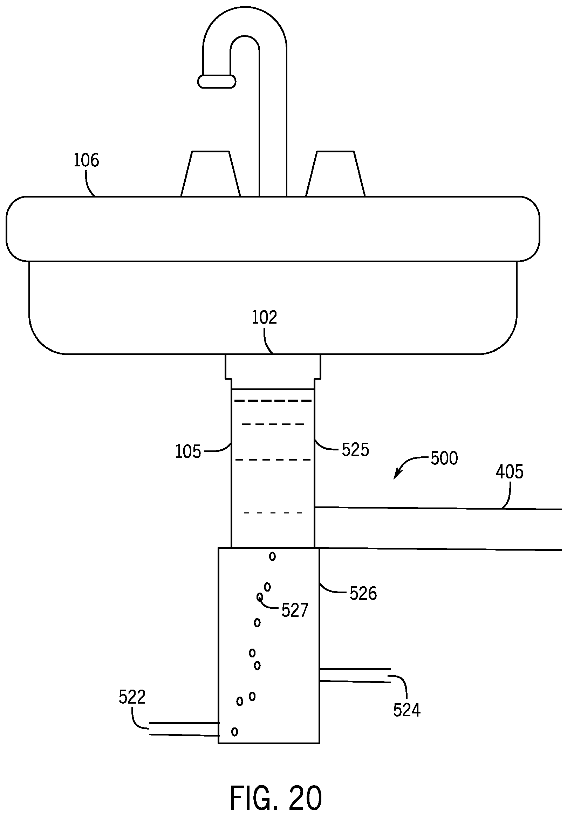

FIG. 20 illustrates an example foam fractionation device 500 for preventing the formation of biofilms or other biological material at a drain 102 of a water appliance such as sink 106. The foam fractionation device 500 may include a liquid pool chamber 526 and a foam chamber 525 located within and/or downstream (e.g., below) the drain pipe 105. In the example of FIG. 20, the liquid pool chamber 526 is below the drain pipe 105 and the foam chamber 525 is within the drain pipe 105.

The foam fractionation device 500 may include at least one inlet and at least one outlet. A connector pipe 404 and/or a P-trap may connect in a Y to the foam fractionation device 500 and the drain pipe 105. In addition, an air inlet 522 may provide gas (e.g., air) to the liquid pool chamber 526. A foam outlet 524 may remove excess foam, liquid, or air from the liquid chamber 526. Additional, different or fewer components may be included.

The foam fractionation device 500 is configured to foam off suspended or dissolved materials from the water in the drain pipe 105 using adsorption on surfaces of one or more bubbles 527. Adsorption is the process in which molecules of solids are adhered to the surface of the one or more bubbles 527. The process may create a film on the surface of the adsorbent. The one of more bubbles 527 float and travel through the liquid pool chamber 526 to float on the water, forming the foam. The foam outlet 521 may include a skimmer to remove the foam. The foam outlet 521 may include suction (e.g., vacuum pressure) to remove the foam. The suction may be provided by a pump. Alternatively, the foam may be removed using a routine of draining and filling the liquid pool chamber.

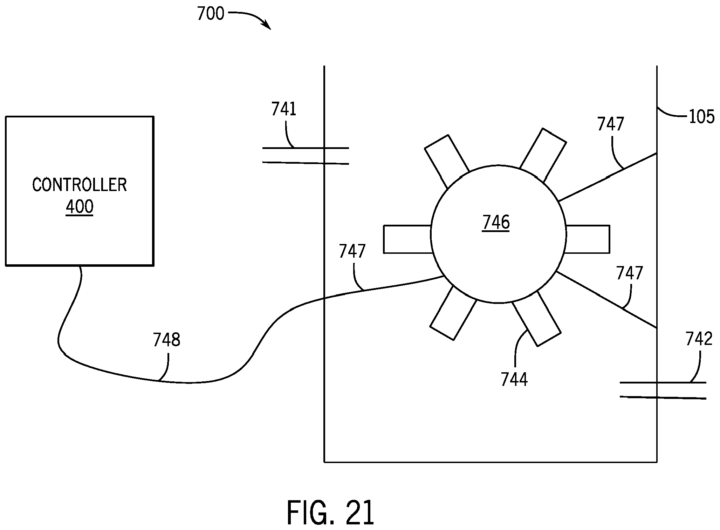

FIG. 21 illustrates an example ultrasonic cavitation device 700. The ultrasonic cavitation device 700 may be coupled to the drain pipe 105 or another locations in the plumbing assembly. The ultrasonic cavitation device 700 may form a separate chamber in the drain pipe 105. The ultrasonic cavitation device 700 may include an inlet 741 and an outlet 742. While not illustrated, one or more transducers may be coupled to a side of the drain pipe 105. Additional, different or fewer components may be included.

The transducers may be ultrasonic transducers that generate high energy waves into the water. The waves may be at a predetermined frequency range that is greater than the range of human hearing. Example frequencies include a range of 20-60 kHz. Specific frequencies or other frequency ranges may be selected for specific implementations. The high energy waves cause compression and expansion on a molecular level and cause the generation of bubbles. As more energy is applied to the bubbles, the ultrasonic cavitation device 700 may cause the bubbles to implode or collapse and release energy. The energy may be applied to any components of the water that are nearby, such as bacteria, biofilm, or other microorganisms. The energy may cause the microorganisms to be broken down into inert particles and/or further into extracellular polymers. The energy may cause local temperature spikes in the water.

The number of transducers may be coupled to the drain pipe 105 or plumbing system may vary. The transducers may be arranged around the tank at a particular height. A round tank may have a set number of transducers around a radius. A square, rectangular, or other polygonal shaped tank may include a transducer on each face. Transducers may be provided at different heights.

The controller 400 may provide electrical power to the transducer through a cable 748. The controller 400 may configured to turn on and off the transducer according to a set pattern. The controller 400 may be able to select the frequency for the transducer. The controller 400 may receive a user selection for the duty cycle or frequency for the transducer. Example duty cycles sufficient to convert high bacteria water to usable for non-potable purposes may be 30-60 minutes.

In addition, or alternatively, a transducer assembly 746, including transducers 744, may be suspended in the middle or substantially the middle of the drain pipe 105. The transducer assembly 146 may be attached to one or more tethers 747. The tethers 747 may include a cable, cord, or other extension member coupled to the wall of the tank by an adhesive or fastener. At least one of the tethers 747 may include the cable 748 for supply power and/or control signals to the transducer assembly 746. The transducer assembly 746 may include any number of transducers 744 pointed toward different portions of the tank 743.

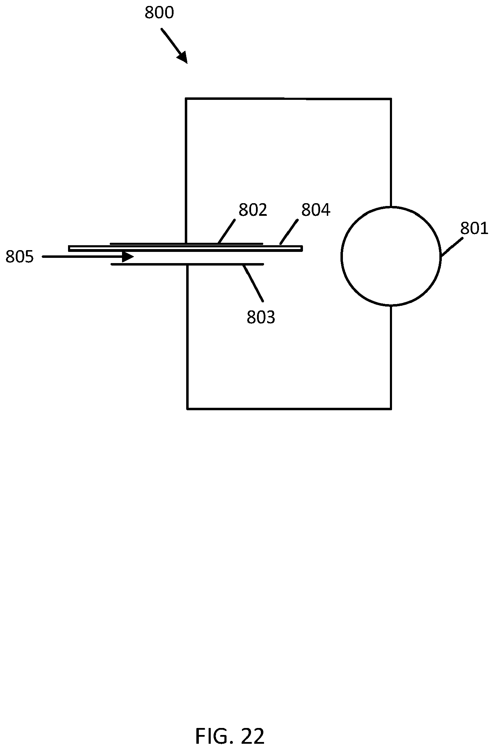

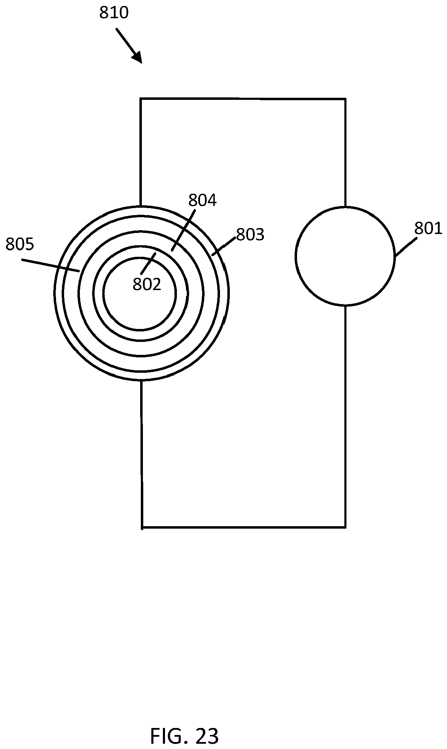

FIG. 22 illustrates an example dielectric barrier discharge device having a planar shape 800. FIG. 23 illustrates an example dielectric barrier discharge device having a cylindrical shape 810. The dielectric barrier discharge device includes a power supply 801,
a high voltage electrode 802, a ground electrode 803, and a dielectric barrier 804 between the high voltage electrode 802 and the ground electrode 803. A discharge gap 805 is between the ground electrode 803 and the dielectric barrier 804 and the high voltage electrode 802. Additional, different, or fewer components may be included.

In FIG. 22, the ground electrode 803, the high voltage electrode 802, and the dielectric barrier 804 are parallel plates. The plates may be planar and square or rectangular in shape. The thicknesses of the electrodes 803 and 802 may be less than 1 millimeter. The thickness of the discharge gap 805 may be 2-5 millimeters. The thicknesses of the dielectric barrier 804 may be about 1 millimeter. The high voltage electrode 802 may be thicker than the ground electrode 803. Thus, in this example, the thickness of the discharge gap 805 may be greater than the thickness of the dielectric barrier 804, which may be greater than the thickness of the high voltage electrode 802, which may be greater than the thickness of the ground electrode 803.

In FIG. 23, the ground electrode 803, the high voltage electrode 802, and the dielectric barrier 804 are coaxial. That is a center of each of the ground electrode 803, the high voltage electrode 802, and the dielectric barrier 804 are the same line or the centers are within a short distance to each other. In some examples, the ground electrode 803, the high voltage electrode 802, and the dielectric barrier 804 are each formed in the shape of a cylinder.

The dielectric barrier discharge device creates a discharge between the electrodes 802 and 803. The discharge may generate ozone. In many examples, the power supply 801 provides an AC current to the dielectric barrier discharge device. The AC current may include a frequency, ranging from low frequencies (10-1000 Hz) to high frequencies (microwave). The high frequency may be modulated on a carrier frequency. When turned on the power supply 801 causes a charge to build on the electrode 802 and eventually a discharge through the dielectric barrier 804 and the air gap 805 to the ground electrode 803.

Example materials for the high voltage electrode 802 may be a conductive material such as steel, copper, silver, brass, platinum, graphite, or gold. Example materials for the ground electrode 803 include any of these materials and may be arranged in a braid, sheet, lattice or other form. Example materials for the dielectric barrier 804 include mica, porcelain, glass, ceramic, or another material.

The power source 801 is configured to provide electrical potential across the high voltage electrode 802 and the ground electrode 803 through the dielectric barrier 804 to generate a plasma from the water. The plasma reduces biofilms in the plumbing device. The plasma may be a non-thermal plasma or cold plasma, having a relatively low temperature to the heavy species (ions and neutrals) that are present.

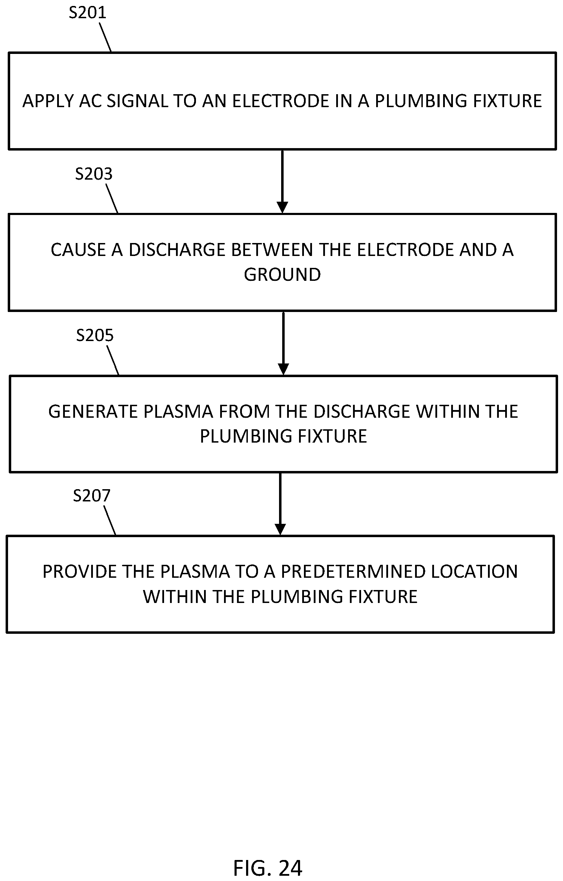

FIG. 24 illustrates a flow chart for the controller 400 of the biofilm prevention system. The acts of the flow chart may be performed by any combination of the control system 400, the network device, or the server. Portions of one or more acts may be performed by the appliance. Additional, different, or fewer acts may be included.

At act S201, the controller 400 applies an AC signal to the high voltage electrode 802. The controller 400 may operate a relay or another switch that activates the power supply 801. In some instances, the user may press a button coupled to the biofilm prevention system that causes the controller 400 to operate the power supply 801. In some examples, the controller 400 may be connected wirelessly and/or via the internet to an external device such as a laptop computer, tablet computer, or a phone. The controller 400 may be connected to a digital assistant that provides commands wirelessly or otherwise to the controller 400.

The controller 400 may configured to turn on the power supply 801 for the biofilm prevention system at certain times or schedules. For example, the biofilm prevention system may be turned on overnight (e.g., once or intermittently between 2AM and 5AM). The biofilm prevention system may be turned on certain days or on a scheduled specified by the user and the external device. The biofilm prevention system may be turned on in response to sensor data according to any of the embodiments herein. For example, the sensor data may include a flow rate of the plumbing device, or an aggregate flow of the plumbing device since the last time the biofilm prevention system was operated. The sensor data may include data indicative of the state of the plumbing device (e.g., a quantity of microorganisms present in the plumbing device or a turbidity of the water in the plumbing device).

The controller 400 may configured to deactivate the biofilm prevention system in case of an error or fault. In some examples, a separate emergency switch may be connected to the high voltage electrode 802 so that the user can immediately disable the biofilm prevention system. In other examples, the controller 400 may monitor a sensor or voltage level in the circuit to determined whether an error has occurred. In response, the controller 400 may generate a fault signal that effectively grounds the high voltage electrode 802 and turns off the biofilm prevention system.

At act S203, the operation of the power supply 801 by the controller 400 causes a discharge between the high voltage electrode 802 and the ground electrode 803. After charge builds up on the high voltage electrode 802 to a level sufficient for a current to jump the gap 805, current will travel to the ground electrode 803. In this process, at act S205, a plasma is discharged. The plasma may include ozone.

At act S207, the controller 400 may provide the plasma to a plumbing device to reduce biofilm in the plumbing device. The controller 400 may open a valve that releases the ozone into the plumbing device. In other examples, when the biofilm prevention device is providing in line in the plumbing device, the ozone is provided continuously, or continuously when the power supply 801 is causing the ozone to be generate.

Processor 300 may be a general purpose or specific purpose processor, an application specific integrated circuit (ASIC), one or more programmable logic controllers (PLCs), one or more field programmable gate arrays (FPGAs), a group of processing components, or other suitable processing components. Processor 300 is configured to execute computer code or instructions stored in memory 352 or received from other computer readable media (e.g., embedded flash memory, local hard disk storage, local ROM, network storage, a remote server, etc.). The processor 300 may be a single device or combinations of devices, such as associated with a network, distributed processing, or cloud computing.

Memory 352 may include one or more devices (e.g., memory units, memory devices, storage devices, etc.) for storing data and/or computer code for completing and/or facilitating the various processes described in the present disclosure. Memory 352 may include random access memory (RAM), read-only memory (ROM), hard drive storage, temporary storage, non-volatile memory, flash memory, optical memory, or any other suitable memory for storing software objects and/or computer instructions. Memory 352 may include database components, object code components, script components, or any other type of information structure for supporting the various activities and information structures described in the present disclosure. Memory 352 may be communicably connected to processor 300 via a processing circuit and may include computer code for executing (e.g., by processor 300) one or more processes described herein. For example, memory 298 may include graphics, web pages, HTML files, XML files, script code, shower configuration files, or other resources for use in generating graphical user interfaces for display and/or for use in interpreting user interface inputs to make command, control, or communication decisions.

In addition to ingress ports and egress ports, the communication interface 353 may include any operable connection. An operable connection may be one in which signals, physical communications, and/or logical communications may be sent and/or received. An operable connection may include a physical interface, an electrical interface, and/or a data interface. The communication interface 353 may be connected to a network. The network may include wired networks (e.g., Ethernet), wireless networks, or combinations thereof. The wireless network may be a cellular telephone network, an 802.11, 802.16, 802.20, or WiMax network, a Bluetooth pairing of devices, or a Bluetooth mesh network. Further, the network may be a public network, such as the Internet, a private network, such as an intranet, or combinations thereof, and may utilize a variety of networking protocols now available or later developed including, but not limited to TCP/IP based networking protocols.

While the computer-readable medium (e.g., memory 352) is shown to be a single medium, the term "computer-readable medium" includes a single medium or multiple media, such as a centralized or distributed database, and/or associated caches and servers that store one or more sets of instructions. The term "computer-readable medium" shall also include any medium that is capable of storing, encoding, or carrying a set of instructions for execution by a processor or that cause a computer system to perform any one or more of the methods or operations disclosed herein.

In a particular non-limiting, exemplary embodiment, the computer-readable medium can include a solid-state memory such as a memory card or other package that houses one or more non-volatile read-only memories. Further, the computer-readable medium can be a random access memory or other volatile re-writable memory. Additionally, the computer-readable medium can include a magnetooptical or optical medium, such as a disk or tapes or other storage device to capture carrier wave signals such as a signal communicated over a transmission medium. A digital file attachment to an e-mail or other self-contained information archive or set of archives may be considered a distribution medium that is a tangible storage medium. Accordingly, the disclosure is considered to include any one or more of a computer-readable medium or a distribution medium and other equivalents and successor media, in which data or instructions may be stored. The computer-readable medium may be non-transitory, which includes all tangible computer-readable media.

In an alternative embodiment, dedicated hardware implementations, such as application specific integrated circuits, programmable logic arrays and other hardware devices, can be constructed to implement one or more of the methods described herein. Applications that may include the apparatus and systems of various embodiments can broadly include a variety of electronic and computer systems. One or more embodiments described herein may implement functions using two or more specific interconnected hardware modules or devices with related control and data signals that can be communicated between and through the modules, or as portions of an application-specific integrated circuit. Accordingly, the present system encompasses software, firmware, and hardware implementations.

An aspect provides a biofilm mitigation apparatus comprising:
a supply container configured to store one or more materials for a formation of a disinfectant;
a disinfectant generator configured to generate the disinfectant from the one or more materials from the supply container;
a power source configured to provide electrical power to the disinfectant generator; and
a delivery tube configured to transport the disinfectant from the disinfectant generator to a biofilm.

The disinfectant may be ozone.

The one or more materials for the formation of the disinfectant may include water.

The one or more materials for the formation of the disinfectant may include gas.

The disinfectant may include hydrogen peroxide, hypochlorous acid, or silver ions.

The delivery tube may be configured to be coupled to a plumbing system.

The plumbing system may be connected to a plurality of appliances and transports the disinfectant to the plurality of appliances.

The plumbing system may include at least one valve configured to release the disinfectant to at least one of the plurality of appliances.

The delivery tube may be configured to be coupled to a downstream side of a tail pipe through a plumbing system.

The delivery tube may be configured to be coupled to an upstream side of a tail pipe through a drain.

An aspect provides a biofilm mitigation apparatus comprising:
a supply container configured to store a biofilm mitigation material;
a pressure generator configured to apply pressure to the biofilm mitigation material; and
a delivery tube configured to transport the biofilm mitigation material from the supply container to a drain under the pressure applied from the pressure generator.

The biofilm mitigation material may be ozone.

The delivery tube may be configured to be coupled to a plumbing system.

The plumbing system may be connected to a plurality of appliances and transports the biofilm mitigation material to the plurality of appliances.

The plumbing system may include at least one valve configured to release the biofilm mitigation material to at least one of the plurality of appliances.

The delivery tube may be configured to be coupled to an upstream side of a tail pipe through a drain.

An aspect provides a biofilm mitigation apparatus comprising:
a supply container configured to store a biofilm mitigation material;
a mixer configured to mix the biofilm mitigation material with water; and
a delivery tube configured to transport the mixture of the biofilm mitigation material and water to a drain.

The biofilm mitigation material may be ozone.

The delivery tube may be configured to be coupled to a plumbing system.

The plumbing system may be connected to a plurality of appliances and transports the biofilm mitigation material to the plurality of appliances.

An aspect provides an internal cleaning system for a drain pipe, the internal cleaning system including:
a robotic scrubber supported on an inside of the drain pipe;
a movable guide member supported on an outside of the drain pipe and configured to guide the robotic scrubber inside of the drain pipe; and
a drive mechanism configured to move the movable guide member in a predetermined path.

The internal cleaning system may comprise:
a controller configured to generate a command for the drive mechanism to move the movable guide member in the predetermined path.

The controller may generate the command for the drive mechanism in response to a user input.

The controller may generate the command for the drive mechanism in response to a wireless signal.

The controller may generate the command for the drive mechanism in response to sensor data.

The sensor data may describe a quantity of biofilm or a condition associated with biofilm.

The predetermined path may include at least one movement in a first direction parallel to a water flow through the drain pipe and at least one movement in a second direction perpendicular to the first direction.

The internal cleaning system may comprise:
a track configured to support the movable guide member in proximity to the drain pipe.

The track may be internal to the drain pipe and may include a tensioning member.

The track may be external to the drain pipe.

The internal cleaning system may comprise:
a manual handle connected to the drive mechanism and configured to move the movable guide member in the predetermined path.

The internal cleaning system may comprise:
a tether between the movable guide member and the drive mechanism.

The internal cleaning system may comprise:
a charging station coupled to the movable guide member and configured to charge a battery of the robotic scrubber.

An aspect provides a drain pipe assembly comprising:
a tail pipe;
a robotic scrubber supported on an inside of the tail pipe;
a movable guide member supported on an outside of the tail pipe and configured to guide the robotic scrubber inside of the pipe; and
a drive mechanism configured to provide to move the movable guide member in a predetermined path.

An aspect provides a control system for an internal cleaning system for a drain pipe, the control system including:
a robotic scrubber supported on an inside of the drain pipe;
a movable guide member supported on an outside of the drain pipe and configured to guide the robotic scrubber inside of the pipe; and
a drive mechanism configured to provide to move the movable guide member in a predetermined path.

An aspect provides a stopper assembly for a drain, the stopper comprising:
a magnet guide including a track and at least one driven magnet slidably engaged with the track of the magnet guide and positioned to provide a magnetic field;
a stopper configured to at least partially seal the drain; and
at least one magnet foot coupled to the stopper and positioned in the magnetic field to move the stopper under a force applied by the magnetic field.

The stopper assembly may comprise:
a drive system for changing a position of the at least one driven magnet, wherein the drive system is configured to receive an input indicative of a stopper position.

The stopper assembly may comprise:
a controller configured to generate the input.

The stopper assembly may comprise:
a user interface electrically coupled to the controller, wherein the input is generated in response to the user interface.

The stopper assembly may comprise:
a timer, wherein the input is generated in response to the timer.

The stopper assembly may comprise:
a sensor, wherein the input is generated in response to the sensor.

The stopper assembly may comprise:
a flow device configured to measure a flow of water, wherein the input is generated in response to the flow device.

The drive system may include a solenoid, a gear, a belt, or another drive member.

The drive system may be configured to move the at least one drive magnet from a first position to a second position where the stopper is moved under the magnetic field to seal the drain.

The drive system may be configured to move the at least one drive magnet from the second position to the first position where the stopper is moved under the magnetic field to unseal the drain.

The at least one driven magnet may include an upper driven magnet above the at least one magnet foot and a lower driven magnet below the at least one magnetic foot.

The at least one magnet foot may include three magnetic feet spaced apart in a radial direction.

The stopper assembly may comprise:
a gasket configured to seal the stopper and the drain.

An aspect provides a disposable sleeve assembly for a drain pipe, the disposable sleeve assembly comprising:
a compartment configured to house a length of disposable sleeve material outside of the drain pipe;
a seal positioned at a first end of the drain pipe and configured to dispense a drain section of the disposable sleeve material into the drain pipe; and
a locking ring positioned at a second end of the drain pipe and configured to couple the drain section of the disposable sleeve material to the drain pipe.

The disposable sleeve assembly may comprise:
a groove configured to receive and support a spent portion of the disposable sleeve material.

The disposable sleeve material may be plastic.

The disposable sleeve assembly may comprise:
an actuator to advance the disposable sleeve material.

The actuator may be manually actuated by a manual input.

The actuator may be actuated based on a timer or a schedule.

The actuator may be actuated based on sensor data.

The disposable sleeve material may prevent biofilm from forming on the drain.

The disposable sleeve assembly may comprise:
a cutting device configured to remove a portion of the disposable sleeve material.

An aspect provides a disposable sleeve assembly for a drain pipe, the disposable sleeve assembly comprising:
a compartment configured to house a length of disposable sleeve material outside off the drain pipe;
a seal positioned at a first end of the drain pipe and configured to dispense a drain section of the disposable sleeve material into the drain pipe;
a drive mechanism configured to advance the drain section of the disposable sleeve material into the drain pipe; and
a controller configured to generate a command for the drive mechanism to advance the drain section of the disposable sleeve material into the drain pipe.

The disposable sleeve assembly may comprise:
a locking ring positioned at a second end of the drain pipe and configured to couple the drain section of the disposable sleeve material to the drain pipe.

The controller may advance the disposable material based on a timer or a schedule.

The controller may advance the disposable material based on sensor data.

The sensor data may describe an environment of the drain pipe, a biofilm formation in the drain pipe, or a usage of the drain pipe.

The disposable sleeve material may prevent biofilm from forming on the drain.

The disposable sleeve assembly may comprise:
a cutting device configured to remove a portion of the disposable sleeve material.

An aspect provides a rotatable plumbing system comprising:
a drain pipe for a water consuming appliance;
a connector pipe connected to a vent pipe;
a first trap section connected to the drain pipe of the water consuming appliance;
a second trap section connected to the first trap section and the connector pipe; and
a drive mechanism configured to move the first trap section or the second trap section relative to the drain pipe or the connector pipe.

The drive mechanism may include a stepper motor or a solenoid.

The first trap section may include a first trap at a first height and the second trap may include a second trap at a second height.

The first trap may hold water at a position of the drive mechanism where the second trap does not hold water.

The drive mechanism may be manually actuated by a manual input.

The drive mechanism may be actuated based on a timer or a schedule.

The drive mechanism may be actuated based on sensor data.

The rotatable plumbing system may comprise:
a controller configured to generate a command to actuate the drive mechanism.

The rotatable plumbing system may comprise:
a rotatable joint between the first trap section and the second trap section.

The rotatable joint may include one or more bearings.

The rotatable plumbing system may comprise:
a third trap section connected between the first trap section and the second trap section.

The rotatable plumbing system may comprise:
a fourth trap section connected between the third trap section and the second trap section.

An aspect provides a method for operation of a dynamic P-trap, the method comprising:
receiving input data for the dynamic P-trap;
generating a rotation command for the dynamic P-trap; and
actuating a drive mechanism in response to the rotation command.

The input data may include a schedule.

The input data may include sensor data.

The rotation command may include a stepper motor angle.

The rotation command may include an electrical current for an electromagnet of a solenoid.

An aspect provides a biofilm prevention device comprising:
at least one chamber coupled to a drain pipe;
at least one inlet for a gas; and
a foam generator to generate foam for the drain pipe.

The foam may adsorb materials from the drain pipe.

The foam generator may be a foam fractionation device.

The biofilm prevention device may comprise:
an outlet for removing excess foam.

An aspect providing a biofilm prevention device comprising:
at least one transducer configured to provide energy to water in a drain pipe; and
a power source electrically coupled to the at least one transducer.

The biofilm prevention device may comprise:
a controller configured to provide commands to the at least one transducer.

The biofilm prevention device may comprise:
a tether coupled to the at least one transducer.

The tether may connect the at least one transducer to the controller.

The tether may connect the at least one transducer to the power source.

The at least one transducer may be configured to generate bubbles.

The at least one transducer may be configured to break down microorganisms in the drain pipe.

The at least one transducer may include at least one ultrasonic transducer.

The at least one transducer may be configured to provide energy to bubbles to attain a volume causing collapse of the bubbles.

An aspect provides a biofilm mitigation apparatus comprising:
a plumbing device including water;
a high voltage electrode;
a ground electrode;
a dielectric barrier between the high voltage electrode and the ground electrode; and
a power source configured to provide electrical potential across the high voltage electrode and the ground electrode through the dielectric barrier to generate a plasma from the water, wherein the plasma reduces biofilms in the plumbing device.

The plasma may be a non-thermal plasma.

The power source may be an alternating current source.

The power source may include a high frequency signal or microwave signal.

The power source may include a modulated high frequency.

The biofilm mitigation apparatus may comprise:
a discharge gap between the ground electrode and the dielectric barrier.

The ground electrode, the high voltage electrode, and the dielectric barrier may be parallel plates.

The ground electrode and the high voltage electrode may be coaxial plates.

The coaxial plates may be cylindrical.

The ground electrode may include a lattice.

The biofilm mitigation apparatus may comprise:
a relay configured to operate the power source.

The biofilm mitigation apparatus may comprise:
a controller configured to cause the power supply to provide a current for the electrical potential across the high voltage electrode and the ground electrode.

The controller may be configured to communicate with a wireless device to receive an instruction for the power supply of the biofilm mitigation apparatus.

The biofilm mitigation apparatus may comprise:
a switch configured to cause the power supply to provide a current for the electrical potential across the high voltage electrode and the ground electrode.

The switch may be electrically coupled to the plumbing device.

An aspect provides a method for biofilm mitigation, the method comprising:
applying an alternating current (AC) to a high voltage electrode;
forming a discharge between the high voltage electrode and a ground electrode;
generating a plasma from the discharge; and
providing the plasma to a plumbing device to reduce biofilm in the plumbing device.

The biofilm mitigation method may comprise:
receiving a command from a user input.

The biofilm mitigation method may comprise:
opening a valve to release the plasma into the plumbing device in response to the user input.

The biofilm mitigation method may comprise:
opening a valve to release the plasma into the plumbing device.

The biofilm mitigation method may comprise:
grounding the high voltage electrode in response to a fault signal.

## Claims

1. A biofilm mitigation apparatus comprising:
a supply container configured to store one or more materials for a formation of a disinfectant;
a disinfectant generator configured to generate the disinfectant from the one or more materials from the supply container;
a power source configured to provide electrical power to the disinfectant generator; and
a delivery tube configured to transport the disinfectant from the disinfectant generator to a biofilm.

2. The biofilm mitigation apparatus of claim 1, wherein the disinfectant is ozone.

3. The biofilm mitigation apparatus of claim 2, wherein the one or more materials for the formation of the disinfectant includes water and/or gas.

4. The biofilm mitigation apparatus of claim 1, claim 2 or claim 3, wherein the disinfectant includes hydrogen peroxide, hypochlorous acid, or silver ions.

5. The biofilm mitigation apparatus of any one of the preceding claims, wherein the delivery tube is configured to be coupled to a plumbing system.

6. The biofilm mitigation apparatus of claim 5, wherein the plumbing system is connected to a plurality of appliances and transports the disinfectant to the plurality of appliances, optionally wherein the plumbing system includes at least one valve configured to release the disinfectant to at least one of the plurality of appliances.

7. The biofilm mitigation apparatus of any one of the preceding claims, wherein the delivery tube is configured to be coupled to a downstream side of a tail pipe through a plumbing system.

8. The biofilm mitigation apparatus of any one of the preceding claims, wherein the delivery tube is configured to be coupled to an upstream side of a tail pipe through a drain.

9. A biofilm mitigation apparatus comprising:
a supply container configured to store a biofilm mitigation material;
a pressure generator configured to apply pressure to the biofilm mitigation material; and
a delivery tube configured to transport the biofilm mitigation material from the supply container to a drain under the pressure applied from the pressure generator.

10. The biofilm mitigation apparatus of claim 9, wherein the delivery tube is configured to be coupled to an upstream side of a tail pipe through a drain.

11. A biofilm mitigation apparatus comprising:
a supply container configured to store a biofilm mitigation material;
a mixer configured to mix the biofilm mitigation material with water; and
a delivery tube configured to transport the mixture of the biofilm mitigation material and water to a drain.

12. The biofilm mitigation apparatus of claim 9, claim 10 or claim 11, wherein the biofilm mitigation material is ozone.

13. The biofilm mitigation apparatus of claim 9, claim 10, claim 11 or claim 12, wherein the delivery tube is configured to be coupled to a plumbing system.

14. The biofilm mitigation apparatus of claim 13, wherein the plumbing system is connected to a plurality of appliances and transports the biofilm mitigation material to the plurality of appliances.

15. The biofilm mitigation apparatus of claim 14, wherein the plumbing system includes at least one valve configured to release the biofilm mitigation material to at least one of the plurality of appliances.
